# EUROPEAN PATENT APPLICATION

(11) **EP 4 592 687 A1**
(43) Date of publication of application: **30.07.2025**
(21) Application number: 25153855.9
(22) Date of filing: 24.01.2025
(51) Int. Cl.: G01N 35/02, G01N 35/04

(54) **SAMPLE ANALYZER AND SAMPLE ASPIRATION METHOD**

(30) Priority: 26.01.2024 CN 202410117040
(71) Applicant: Shenzhen New Industries Biomedical Engineering Co., Ltd., Shenzhen, Guangdong 518122 (CN)
(72) Inventor: LIU, Yuhuai, Shenzhen, 518122 (CN); LIN, Ronghua, Shenzhen, 518122 (CN); YANG, Zhenning, Shenzhen, 518122 (CN); KANG, Lele, Shenzhen, 518122 (CN); HUANG, Qifeng, Shenzhen, 518122 (CN); ZHENG, Zijia, Shenzhen, 518122 (CN); LI, Jia, Shenzhen, 518122 (CN)
(74) Representative: Metida

(57) **Abstract**

The present invention discloses a sample analyzer and a sample aspiration method. The sample analyzer includes a first track (21), a second track (22), a reaction wheel (10), an electrolyte analyzing mechanism (30), a first sample dispensing mechanism, and a second sample dispensing mechanism. In the present invention, the first sample dispensing mechanism independently transfers the samples requiring biochemical analyzing from the first track (21) to the reaction vessels carried by the reaction wheel (10), and the second sample dispensing mechanism independently transfers the samples requiring electrolyte analyzing from the second track (22) to the analyzing vessels carried by the electrolyte analyzing mechanism (30).

## Description

### TECHNICAL FIELD

The present invention relates to the technical field of analytical instruments, and in particular to a sample analyzer and a sample aspiration method.

### BACKGROUND

Biochemical analyzers, immunoassay analyzers, and cell analyzers are all sample analyzers that are designed for quantitative and qualitative analysis of samples.

With the increasing multifunctionality of sample analyzers, in addition to performing liver function tests, kidney function tests, fasting blood glucose tests, uric acid tests, and other tests on the reaction wheel, electrolyte analyzing mechanisms are often integrated in the sample analyzers for electrolyte analyzing.

In typical sample analyzers, those equipped with integrated electrolyte analyzing mechanisms face the challenge of a decreased efficiency of supplying samples to the reaction wheel caused by balancing the sample supply required by the electrolyte analyzing mechanism, which results in affecting the analyzing efficiency of the reaction wheel.

### SUMMARY

The main objective of the present invention is to provide a sample analyzer and a sample aspiration method which can solve, or at least partially alleviate the above shortcoming in prior art.

In some embodiments, the present invention provides a sample analyzer, including:
a first track;
a second track;
a reaction wheel carrying a plurality of reaction vessels;
an electrolyte analyzing mechanism carrying one or more analyzing vessels;
a first sample dispensing mechanism configured to transfer samples requiring biochemical analyzing located on the first track into the plurality of reaction vessels; and
a second sample dispensing mechanism configured to transfer samples requiring electrolyte analyzing located on the second track into the one or more analyzing vessels.

In some embodiments, the reaction wheel has a horizontal symmetry line and a vertical symmetry line; and
the electrolyte analyzing mechanism, a first rotation center of the second sample dispensing mechanism and the second track are arranged between the first track and the horizontal symmetry line and are located on a same side of the vertical symmetry line.

In some embodiments, when the second sample dispensing mechanism transfers the samples requiring electrolyte analyzing from the second track to the one or more analyzing vessels, a second sample rack remains stationary on the second track, and the second sample dispensing mechanism moves to different sample containers carried by the second sample rack to aspirate the samples requiring electrolyte analyzing.

In some embodiments, the second sample dispensing mechanism includes a folding arm and a sample needle, and the folding arm is configured to drive the sample needle to move to the different sample containers carried by the second sample rack to aspirate the samples requiring electrolyte analyzing; or
wherein the second sample dispensing mechanism includes a telescopic arm and a sample needle, and the telescopic arm is configured to drive the sample needle to move to the different sample containers carried by the second sample rack to aspirate the samples requiring electrolyte analyzing.

In some embodiments, the first sample dispensing mechanism includes a first sample delivery arm and a second sample delivery arm which are independent from each other, the first sample delivery arm is rotatable around a second rotation center, and the second sample delivery arm is rotatable around a third rotation center; and
wherein the first rotation center and the second rotation center are respectively located on opposite sides of the vertical symmetry line, the third rotation center and the second rotation center are located on a same side of the vertical symmetry line, and the second rotation center is located between the first rotation center and the third rotation center.

In some embodiments, the reaction wheel includes an outer reaction ring, and an inner reaction ring located on an inner side of the outer reaction ring;
wherein the first sample delivery arm is configured to transfer the samples requiring biochemical analyzing located on the first track into reaction vessels carried by the inner reaction ring, and the second sample delivery arm is configured to transfer the samples requiring biochemical analyzing located on the first track into reaction vessels carried by the outer reaction ring; and
wherein both the first sample delivery arm and the second sample delivery arm perform aspiration at a shared sample aspiration position on the first track, and a first sample rack moves along the first track to make a plurality of sample containers on the first sample rack sequentially reach the shared sample aspiration position.

In some embodiments, the reaction wheel includes a first reagent dispensing position and a second reagent dispensing position;
the sample analyzer further includes:
a reagent container storage mechanism configured to carry a plurality of reagent containers, wherein the reagent container storage mechanism is located on an outside of the reaction wheel and on a side of the horizontal symmetry line away from the electrolyte analyzing mechanism, and the reagent container storage mechanism includes a first reagent aspirating position and a second reagent aspirating position; and
a reagent injection mechanism including a first reagent needle and a second reagent needle which are independently controlled, wherein the first reagent needle is configured to draw a reagent from one of the plurality of reagent containers that is located at the first reagent aspirating position, transfer the reagent along a first straight line, and inject the reagent into one of the plurality of reaction vessels that is located at the first reagent dispensing position at a time, and the second reagent needle is configured to draw a reagent from one of the plurality of reagent containers that is located at the second reagent aspirating position, transfer the reagent along a second straight line, and inject the reagent into one of the plurality of reaction vessels that is located at the second reagent dispensing position at a time.

In some embodiments, samples located on the first track and samples located on the second track enter the sample analyzer via the first track.

In some embodiments, a sample only to be transferred to one of the plurality of reaction vessels is received by a sample container carried by a first sample rack;
a sample at least to be transferred to the analyzing vessel or one of the analyzing vessels is received by a sample container carried by a second sample rack; and
the sample analyzer further includes a sample rack transfer mechanism, and the second sample rack is transferable from the first track to the second track by the sample rack transfer mechanism.

In some embodiments, the sample analyzer includes a third track, wherein:
the first track, the second track and the third track are arranged parallel to each other, and the first sample rack and the second sample rack exit the sample analyzer via the third track; and
the sample rack transfer mechanism is further configured to transfer the first sample rack on the first track or the second sample rack on the second track to the third track.

In some embodiments, the sample rack transfer mechanism includes a transfer track and a first driving device, and the first driving device is configured to drive the transfer track to connect to a first discharge position of the first track and further configured to drive the transfer track to connect to either an access position of the second track or a third feed position of the third track; or
wherein the sample rack transfer mechanism includes a second driving device and a gripper, and wherein the gripper driven by the second driving device is configured to transfer the second sample rack on the first track to the second track; and transfer the second sample rack on the second track or the first sample rack on the first track to the third track.

In some embodiments, the first track is configured to transfer both the first sample rack and the second sample rack, the first sample rack carries samples that require biochemical analyzing, and the second sample rack carries samples that require electrolyte analyzing only or require both electrolyte analyzing and biochemical analyzing; and
the second sample rack is transferable from the first track to the second track and carries samples that require electrolyte analyzing.

In some embodiments, the sample analyzer is configured for:
delivering a sample rack into the sample analyzer from a first feed position of the first track;
transferring, by the first sample dispensing mechanism, the samples requiring biochemical analyzing from a plurality of sample containers carried by the sample rack to the plurality of reaction vessels;
transferring the sample rack from a first discharge position of the first track to a sample rack transfer mechanism;
transferring the sample rack from the sample rack transfer mechanism to an access position of the second track;
transferring the sample rack from the access position to a second sampling area of the second track;
transferring, by the second sample dispensing mechanism, the samples requiring electrolyte analyzing from the plurality of sample containers carried by the sample rack to the one or more analyzing vessels carried by the electrolyte analyzing mechanism; and
transferring the sample rack from the second sampling area to the access position and then from the access position back to the sample rack transfer mechanism after the step of transferring, by the second sample dispensing mechanism, the samples requiring electrolyte analyzing from the plurality of sample containers carried by the sample rack to the one or more analyzing vessels carried by the electrolyte analyzing mechanism.

In some embodiments, for delivering a sample rack into the sample analyzer from a first feed position of the first track, the sample analyzer is further configured for:
transferring the sample rack from the first feeding end to a first sampling area of the first track, with the plurality of sample containers carried by the sample rack sequentially passing through a shared sample aspiration position;
wherein for transferring, by the first sample dispensing mechanism, the samples requiring biochemical analyzing from a plurality of sample containers carried by the sample rack to the plurality of reaction vessels, the sample analyzer is further configured for:
transferring, by the first sample dispensing mechanism, the samples requiring biochemical analyzing in the plurality of sample containers at the shared sample aspiration position to the plurality of reaction vessels carried by the reaction wheel during a process of the plurality of sample containers carried by the sample rack sequentially passing through the shared sample aspiration position; and
transferring the sample rack from the first sampling area to the first discharge position after transferring, by the first sample dispensing mechanism, the samples requiring biochemical analyzing in the plurality of sample containers at the shared sample aspiration position to the plurality of reaction vessels carried by the reaction wheel.

In some embodiments, the first sample dispensing mechanism includes a first sample delivery arm and a second sample delivery arm which are independent from each other; and
the reaction wheel includes an outer reaction ring and an inner reaction ring located on an inner side of the outer reaction ring; and
wherein for transferring, by the first sample dispensing mechanism, the samples requiring biochemical analyzing in the plurality of sample containers at the shared sample aspiration position to the plurality of reaction vessels, the sample analyzer is further configured for:
aspirating, by the first sample delivery arm, a first sample in one of the plurality of sample containers at the shared sample aspiration position;
transferring, by the first sample delivery arm, the first sample to one of the plurality of reaction vessels carried by the inner reaction ring; and
aspirating and transferring, by the second sample delivery arm, the first sample in the one of the plurality of sample containers at the shared sample aspiration position or a second sample in another one of the plurality of sample containers at the shared sample aspiration position to one of the plurality of reaction vessels carried by the outer reaction ring when the first sample is transferred by the first sample delivery arm to the one of the plurality of reaction vessels carried by the inner reaction ring.

In some embodiments, after transferring the sample rack from the second sampling area to the access position and then from the access position back to the sample rack transfer mechanism, the sample analyzer is further configured for:
driving, by a first driving device of the sample rack transfer mechanism, a transfer track to connect to a third feed position of a third track;
transferring the sample rack from the transfer track to the third feed position; and
transferring the sample rack from the third feed position to a third discharge position of the third track to make the sample rack exit the sample analyzer.

In some embodiments, the sample analyzer is further configured for:
in a first sampling area, transferring, by the first sample dispensing mechanism, the samples requiring biochemical analyzing from a plurality of sample containers carried by a first sample rack to the plurality of reaction vessels carried by the reaction wheel; and
in a second sampling area, transferring, by the second sample dispensing mechanism, the samples requiring electrolyte analyzing from a plurality of sample containers carried by a second sample rack to the one or more analyzing vessels carried by the electrolyte analyzing mechanism,
wherein before transferring the samples requiring electrolyte analyzing from the plurality of sample containers carried by the second sample rack to the one or more analyzing vessels carried by the electrolyte analyzing mechanism, the sample analyzer is further configured for transferring the second sample rack to the second sampling area, during which the second sample rack passes through the first sampling area.

In some embodiments, the first sampling area is located on the first track, the second sampling area is located on the second track, and the second track is parallel to the first track;
the sample analyzer is further configured for:
transferring the second sample rack from the first track to a sample rack transfer mechanism; and
transferring the second sample rack from the sample rack transfer mechanism to the second track.

In some embodiments, the sample analyzer is further configured for:
transferring, by the first sample dispensing mechanism, the samples requiring biochemical analyzing from the plurality of sample containers carried by the second sample rack to the plurality of reaction vessels when the second sample rack reaches the first sampling area; and
transferring, by the second sample dispensing mechanism, the samples requiring electrolyte analyzing from the plurality of sample containers carried by the second sample rack to the one or more analyzing vessels carried by the electrolyte analyzing mechanism when the second sample rack reaches the second sampling area.

In some embodiments, for transferring, by the first sample dispensing mechanism, the samples requiring biochemical analyzing from a plurality of sample containers carried by a first sample rack to the plurality of reaction vessels, the plurality of sample containers carried by the first sample rack pass sequentially through a shared sample aspiration position, and the first sample dispensing mechanism sequentially transfers the samples requiring biochemical analyzing from the plurality of sample containers located at the shared sample aspiration position to the plurality of reaction vessels.

In some embodiments, the present invention further provides a sample aspiration method for a sample analyzer, including:
in a first sampling area, transferring, by a first sample dispensing mechanism, samples requiring biochemical analyzing from a plurality of sample containers carried by a first sample rack to a plurality of reaction vessels carried by a reaction wheel; and
in a second sampling area, transferring, by a second sample dispensing mechanism, samples requiring electrolyte analyzing from a plurality of sample containers carried by a second sample rack to one or more analyzing vessels carried by a electrolyte analyzing mechanism,
wherein before the step of transferring samples requiring electrolyte analyzing from a plurality of sample containers carried by a second sample rack to one or more analyzing vessels carried by a electrolyte analyzing mechanism, the method further includes transferring the second sample rack to the second sampling area, during which the second sample rack passes through the first sampling area.

In some embodiments, the first sampling area is located on a first track, the second sampling area is located on a second track, and the second track is parallel to the first track;
the method further includes steps of:
transferring the second sample rack from the first track to a sample rack transfer mechanism; and
transferring the second sample rack from the sample rack transfer mechanism to the second track.

In some embodiments, the sample aspiration method for the sample analyzer further includes steps of:
transferring, by the first sample dispensing mechanism, the samples requiring biochemical analyzing from the plurality of sample containers carried by the second sample rack to the plurality of reaction vessels when the second sample rack reaches the first sampling area; and
transferring, by the second sample dispensing mechanism, the samples requiring electrolyte analyzing from the plurality of sample containers carried by the second sample rack to the one or more analyzing vessels carried by the electrolyte analyzing mechanism when the second sample rack reaches the second sampling area;
wherein after the step of transferring, by the first sample dispensing mechanism, the samples requiring biochemical analyzing from the plurality of sample containers carried by the second sample rack to the plurality of reaction vessels, the method further includes:
transferring the second sample rack from the first sampling area to a first discharge position of the first track, and then from the first discharge position to the sample rack transfer mechanism;
transferring, by the sample rack transfer mechanism, the second sample rack to an access position of the second track;
transferring, the second sample rack from the access position to the second sampling area, that is followed by the step of transferring, by the second sample dispensing mechanism, the samples requiring electrolyte analyzing from the plurality of the sample containers carried by the second sample rack to the one or more analyzing vessels carried by the electrolyte analyzing mechanism; and
transferring the second sample rack from the second sampling area to the access position, and then from the access position to the sample rack transfer mechanism after the step of transferring, by the second sample dispensing mechanism, the samples requiring electrolyte analyzing from the plurality of sample containers carried by the second sample rack to the one or more analyzing vessels.

In some embodiments, during the step of transferring, by a second sample dispensing mechanism, samples requiring electrolyte analyzing from a plurality of the sample containers carried by a second sample rack to one or more analyzing vessels, the second sample rack remains stationary in the second sampling area, and the second sample dispensing mechanism is capable of moving to different sample containers carried by the second sample rack to aspirate the samples requiring electrolyte analyzing.

In some embodiments, during the step of transferring, by a first sample dispensing mechanism, samples requiring biochemical analyzing from a plurality of sample containers carried by a first sample rack to a plurality of reaction vessels, the plurality of sample containers carried by the first sample rack pass sequentially through a shared sample aspiration position, and the first sample dispensing mechanism sequentially transfers the samples requiring biochemical analyzing from a plurality of sample containers located at the shared sample aspiration position to the plurality of reaction vessels;
wherein the first sample dispensing mechanism includes a first sample delivery arm and a second sample delivery arm which are independent from each other; and
the reaction wheel includes an outer reaction ring and an inner reaction ring located on an inner side of the outer reaction ring; and
wherein the step of transferring, by the first sample dispensing mechanism, the samples requiring biochemical analyzing from a plurality of sample containers at the shared sample aspiration position to the plurality of reaction vessels includes steps of:
aspirating, by the first sample delivery arm, a first sample in one of the plurality of sample containers at the shared sample aspiration position;
transferring, by the first sample delivery arm, the first sample to one of the plurality of reaction vessels carried by the inner reaction ring; and
aspirating and transferring, by the second sample delivery arm, the first sample in the one of the plurality of sample containers at the shared sample aspiration position or a second sample in another one of the plurality of sample containers at the shared sample aspiration position to one of the plurality of reaction vessels carried by the outer reaction ring when the first sample is transferred by the first sample delivery arm to one of the plurality of reaction vessels carried by the inner reaction ring.

In the present invention, the first sample dispensing mechanism independently transfers the samples requiring biochemical analyzing from the first track to the reaction vessels carried by the reaction wheel, and the second sample dispensing mechanism independently transfers the samples requiring electrolyte analyzing from the second track to the analyzing vessels carried by the electrolyte analyzing mechanism. This enables the efficient transfer of samples required for electrolyte analyzing without affecting the transfer of samples into the reaction vessels carried by the reaction wheel.

### BRIEF DESCRIPTION OF THE DRAWINGS

The accompanying drawings described herein are provided to facilitate the understanding of the present invention and constitute a part of this invention. The illustrative embodiments and their descriptions are intended to explain the present invention and should not be construed as undue limitation thereto. In the drawings:
FIG. 1 is an overall schematic structural view of a sample analyzer according to one embodiment of the present invention;
FIG. 2 is a schematic structural view partially illustrate a reagent injection mechanism according to one embodiment of the present invention;
FIG. 3 is a schematic flowchart of a sample transportation method according to one embodiment of the present invention;
FIG. 4 is a schematic flowchart of a sample transportation method according to another embodiment of the present invention; and
FIG. 5 is a schematic flowchart of a sample aspiration method according to one embodiment of the present invention.

### DESCRIPTION OF THE EMBODIMENTS

It should be noted that, where there is no conflict, the embodiments and features of the embodiments in the present invention can be combined with each other. The following detailed descriptions of the present invention will be made with reference to the accompanying drawings and in conjunction with the embodiments.

It should be noted that the terms used here are merely for describing specific embodiments only, and are not intended to limit the exemplary embodiments of the present invention. Furthermore, it should be understood that when the terms "comprise" as well as its variants and/or "include" as well as its variants are used in this description, they indicate the presence of features, steps, operations, devices, components, and/or their combinations.

Unless otherwise specified, the relative arrangement, numerical expressions, and values of the components and steps described in these embodiments do not limit the scope of the present invention. Additionally, it should be understood that for the sake of convenience in description, the sizes of the parts shown in the drawings are not drawn to scale. Technologies, methods, and devices known to those skilled in the art of the relevant field may not be discussed in detail, but where appropriate, these technologies, methods, and devices should be regarded as part of the invention. In all the examples shown and discussed here, any specific values should be interpreted as merely exemplary, and not as limitations. Therefore, other examples of the exemplary embodiments may have different values. It should be noted that similar reference numerals and letters in the following drawings represent similar items. Therefore, once an item is defined in one drawing, it does not need to be further discussed in subsequent drawings.

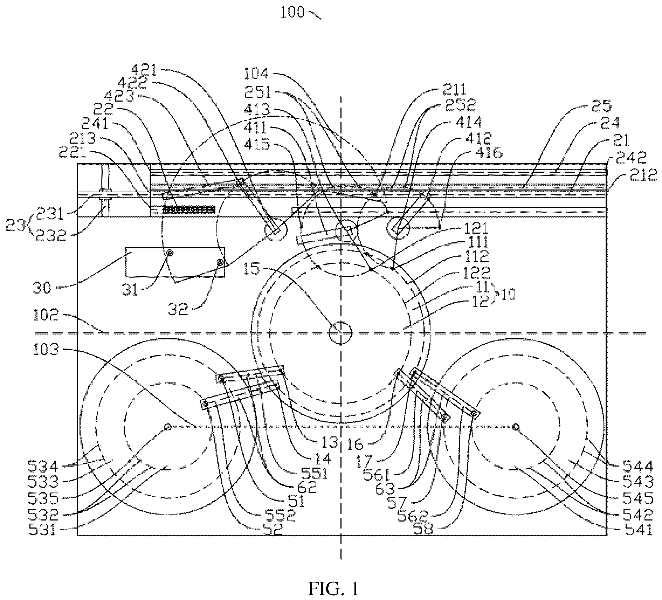

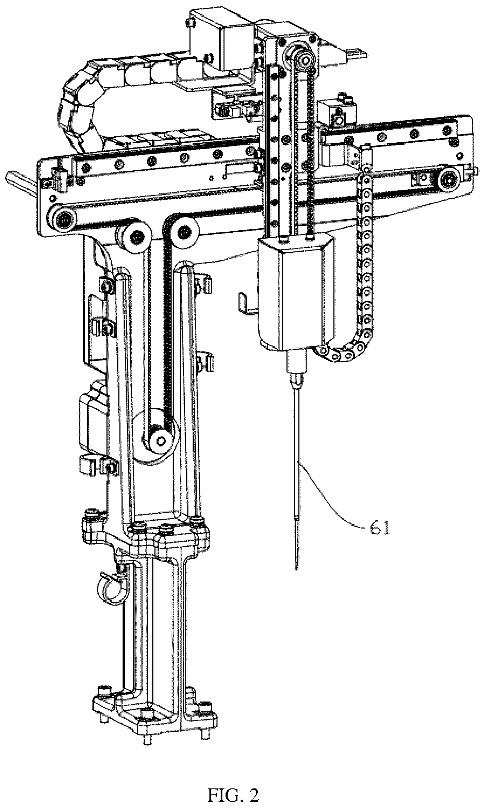

Referring to FIG. 1 to FIG. 2, The present invention provides a sample analyzer 100, which includes a first track 21, a second track 22, a reaction wheel 10, an electrolyte analyzing mechanism 30, a first sample dispensing mechanism, and a second sample dispensing mechanism.

The reaction wheel 10 is provided on a support frame and carries a plurality of reaction vessels. The reaction vessels are used to receive samples that require biochemical analyzing.

The electrolyte analyzing mechanism 30 is provided on the support frame, located outside the reaction wheel 10. The electrolyte analyzing mechanism 30 carries one or more analyzing vessels, which are used to receive samples that require electrolyte analyzing.

The first sample dispensing mechanism is provided on the support frame, outside the reaction wheel 10. It is used to transfer samples from the first track 21 to the reaction vessels for biochemical analyzing.

The second sample dispensing mechanism is provided on the support frame, outside the reaction wheel 10. It is used to transfer samples from the second track 22 to the analyzing vessels for electrolyte analyzing.

In this embodiment, the second sample dispensing mechanism independently transfers the samples requiring electrolyte analyzing from the second track 22 to the analyzing vessels carried by the electrolyte analyzing mechanism 30, and the first sample dispensing mechanism independently transfers the samples requiring biochemical analyzing from the first track 21 to the reaction vessels carried by the reaction wheel 10. This makes the samples transfer to the analyzing vessels independently from to the reaction vessels, thereby enabling the efficient transfer of samples required for electrolyte analyzing without affecting the transfer of samples into the reaction vessels carried by the reaction wheel 10.

In some embodiments, the reaction wheel 10 is provided with a sample dispensing position. The first sample dispensing mechanism draws samples requiring biochemical analyzing from the first track 21 and transfers them to the reaction vessels located at the sample dispensing position.

In some embodiments, the reaction wheel 10 can carry multiple reaction vessels. The reaction wheel 10 may rotate to move the multiple reaction vessels along a third rotation path. The multiple reaction vessels sequentially pass through the sample dispensing position when they rotate along the third rotation path.

In some embodiments, the reaction wheel 10 is provided with a horizontal symmetry line 102 and a vertical symmetry line 104 that pass through the third rotation center 15. The horizontal symmetry line 102 and the vertical symmetry line 104 divide the reaction wheel 10 into the first, second, third, and fourth quadrants.

The electrolyte analyzing mechanism 30, the first rotation center 421 of the second sample dispensing mechanism, and the second track 22 are positioned between the first track 21 and the horizontal symmetry line 102, and are located on the same side of the vertical symmetry line 104. This arrangement integrates the electrolyte module, which includes the electrolyte analyzing mechanism 30, the second sample dispensing mechanism, and the second track 22, into a limited space on the support frame. As a result, the sample analyzer 100 is able to efficiently perform biochemical analysis while simultaneously conducting electrolyte analyzing efficiently in a limited pace.

In one embodiment, the electrolyte module is positioned on the outside of the reaction wheel 10 and located in the first quadrant.

In another embodiment, the electrolyte module is positioned on the outside of the reaction wheel 10 and located in the second quadrant.

In other embodiments, the electrolyte module may also be positioned on the outside of the reaction wheel 10 and located in the third quadrant, or on the outside of the reaction wheel 10 and located in the fourth quadrant.

In some embodiments, a sample only to be transferred to one of the plurality of reaction vessels is received by a sample container carried by a first sample rack. That is, after the samples have been completely aspirated by means of the first sample dispensing mechanism from the sample containers carried by the first sample rack, the first sample rack is directly recycled.

A sample at least to be transferred to the analyzing vessel or one of the analyzing vessels is received by a sample container carried by a second sample rack. That is, after the samples have been completely aspirated by means of the second sample dispensing mechanism from the sample containers carried by the second sample rack, the second sample rack is then recycled.

In one embodiment, the sample needle of the second sample dispensing mechanism and the second track 22 are intersected with each other at an intersection that is configured as an electrolyte sampling point. The sample needle of the second sample dispensing mechanism aspires samples from the sample containers at the electrolyte sampling point.

When the second sample dispensing mechanism transfers the samples requiring electrolyte analyzing from the second track 22 to the analyzing vessels, that is, when the second sample dispensing mechanism transfers the samples from the sample containers carried by the second sample rack to the analyzing vessels, the movement of the second track 22 drives each sample container carried by the second sample rack to pass through the electrolyte sampling point sequentially, allowing the sample needle of the second sample dispensing mechanism to aspirate the samples.

Specifically, the sample needle of the second sample dispensing mechanism rotates around the first rotation center 421, and reciprocate between the electrolyte sampling point and the analyzing vessel to transfer samples.

In another embodiment, because there is a limited mounting space on the second track 22, the second sample rack on the second track 22 is unable to move, such that the sample containers on the second track 22 fail to sequentially pass through the electrolyte sampling point.

Therefore, after the second sample rack is transported to the second track 22, the second sample rack remains stationary on the second track 22, and the second sample dispensing mechanism can move to aspirate sample from the respective sample containers carried by the second sample rack.

Specifically, the sample needle of the second sample dispensing mechanism can move to aspirate sample from the respective sample containers carried by the second sample rack, which remains stationary on the second track 22.

In one embodiment, the second sample dispensing mechanism includes a folding arm and a sample needle. The folding arm can move the sample needle to aspirate sample from the respective sample containers carried by the second sample rack.

Specifically, the folding arm includes at least a first folding sub-arm 422 and a second folding sub-arm 423. One end of the first folding sub-arm 422 is rotatably provided about the first rotation center 421, and the other end of the first folding sub-arm 422 is connected to one end of the second folding sub-arm 423. The second folding sub-arm 423 can rotate relative to the first folding sub-arm 422, and the other end of the second folding sub-arm 423 is connected to the sample needle.

By providing the sample needle on the folding arm, the sample needle may aspirate sample within the second sampling area on the second track 22. The second sampling area may accommodate at least one of the second sample racks, allowing the sample needle to aspirate sample from any sample container in the second sample rack that is stationarily positioned in the second sampling area.

In another embodiment, the second sample dispensing mechanism includes a telescopic arm and a sample needle. The telescopic arm may move the sample needle to aspirate sample from the respective sample containers carried by the second sample rack.

Specifically, the telescopic arm includes at least a first telescopic sub-arm and a second telescopic sub-arm. One end of the first telescopic sub-arm is rotatably provided about the first rotation center 421, and the other end of the first telescopic sub-arm is connected to one end of the second telescopic sub-arm. The second telescopic sub-arm is telescopic relative to the first telescopic sub-arm, and the other end of the second telescopic sub-arm is connected to the sample needle.

By providing the sample needle on the telescopic arm, the sample needle may aspirate sample within the second sampling area on the second track 22. The second sampling area may accommodate at least one of the second sample racks, allowing the sample needle to aspirate sample from any sample container in the second sample rack that is stationarily positioned in the second sampling area.

In some embodiment, the electrolyte analyzing mechanism 30 includes at least one sample injection position for adding electrolyte analyzing samples.

In a specific embodiment, the electrolyte analyzing mechanism 30 includes a first sample injection position 31 and a second sample injection position 32. The second sample dispensing mechanism can transfer the sample to the analyzing vessels at either the first sample injection position 31 or the second sample injection position 32.

In some embodiment, the first sample dispensing mechanism includes a first sample delivery arm 411 and a second sample delivery arm 412 that are independent from each other. The first sample delivery arm 411 and the second sample delivery arm 412 are each used to transfer samples from the sample containers carried by the first sample rack on the first track 21 to the reaction vessels carried by the reaction wheel 10.

In one embodiment, the sample needle on the first sample delivery arm 411 and the sample needle on the second sample delivery arm 412 respectively move in a straight line to perform the sample transfer.

In another embodiment, the first sample delivery arm 411 rotates around the second rotation center 413, and the second sample delivery arm 412 rotates around the third rotation center 414.

In some embodiments, the reaction wheel 10 has a single-ring structure. The reaction wheel 10 is provided with multiple third carrying positions along the third rotation path, each of the third carrying positions can carry one reaction vessel. The reaction wheel 10 can rotate to move the multiple third carrying positions to sequentially pass through the sample dispensing position.

In another embodiment, the reaction wheel 10 has a dual-ring structure, meaning that the reaction wheel 10 includes an outer reaction ring 11 and an inner reaction ring 12 located inside the outer reaction ring 11.

The sample needle of the first sample delivery arm 411 is used to transfer the sample from the first track 21 to the reaction vessels carried by the inner reaction ring 12, while the sample needle of the second sample delivery arm 412 is used to transfer the sample from the first track 21 to the reaction vessels carried by the outer reaction ring 11.

In some embodiments, the inner reaction ring 12 is provided with an inner sample dispensing position 121, while the outer reaction ring 11 is provided with an outer sample dispensing position 111.

The inner reaction ring 12 is provided with multiple third inner carrying positions 122 along a third internal rotation path, and each of the third inner carrying positions 122 can carry a reaction vessel. The inner reaction ring 12 can rotate to move the multiple third inner carrying positions 122 to sequentially pass through the inner sample dispensing position 121.

The outer reaction ring 11 is provided with multiple third outer carrying positions 112 along a third external rotation path, and each of the third outer carrying positions 112 can carry a reaction vessel. The outer reaction ring 11 can rotate to move the multiple third outer carrying positions 112 to sequentially pass through the outer sample dispensing position 111.

In some embodiments, both the first sample delivery arm 411 and the second sample delivery arm 412 aspirate sample at a shared sample aspiration position 211 on the first track 21. The first sample rack moves along the first track 21 so that the sample containers in the first sample rack sequentially reach the shared sample aspiration position 211.

In some embodiments, the movement trajectory of the sample needle on the first sample delivery arm 411 and the movement trajectory of the sample needle on the second sample delivery arm 412 both intersect with the first track 21 at the shared sample aspiration position 211, so that both the sample needle of the first sample delivery arm 411 and the sample needle of the second sample delivery arm 412 aspirate sample at the shared sample aspiration position 211.

By providing the shared sample aspiration position 211, the sample containers carried by the first sample rack pass through the shared sample aspiration position 211 sequentially, allowing the sample containers to be aspirated by the sample needle of either the first sample delivery arm 411 or the second sample delivery arm 412. This effectively simplifies the movement path of the first sample rack on the first track 21 and reduces the complexity of controlling the movement of the first sample rack on the first track 21. Additionally, it improves the transportation and sampling efficiency of the sample containers.

Moreover, by providing the shared sample aspiration position 211, which can also serve as a reference for calibrating the movement paths of the sample needles of the first sample delivery arm 411 and the second sample delivery arm 412. This improves calibration efficiency and enables the first sampling arm 411 and the second sampling arm 412 to be positioned adjacent to each other, thereby reducing the space occupied by the first sample delivery arm 411 and the second sample delivery arm 412, and increasing the integration level of the first sample delivery arm 411 and the second sample delivery arm 412.

In case of the reaction wheel 10 with the single-ring structure, the sample needle of the first sample delivery arm 411 and the sample needle of the second sample delivery arm 412 respectively transfer the samples taken from the shared sample aspiration position 211 to the reaction vessels carried by the reaction wheel 10.

In case of the reaction wheel 10 with the dual-ring structure, the sample needle of the first sample delivery arm 411 can be used to transfer the sample taken from the shared sample aspiration position 211 to the reaction vessels carried by either the inner reaction ring 12 or the outer reaction ring 11. The sample needle of the second sample delivery arm 412 can also be used to transfer the samples taken from the shared sample aspiration position 211 to the reaction vessels carried by either the inner reaction ring 12 or the outer reaction ring 11.

Preferably, the sample needle of the first sample delivery arm 411 is used to transfer the samples taken from the shared sample aspiration position 211 to the reaction vessels carried by the inner reaction ring 12, and the sample needle of the second sample delivery arm 412 is used to transfer the samples taken from the shared sample aspiration position 211 to the reaction vessels carried by the outer reaction ring 11.

In some embodiments, the first rotation center 421 and the second rotation center 413 are respectively located on opposite sides of the vertical symmetry line 104, and the third rotation center 414 and the second rotation center 413 are located on the same side of the vertical symmetry line 104. This arrangement ensures that the first sample dispensing mechanism and the second sample dispensing mechanism are placed on opposite sides of the vertical symmetry line 104, thus preventing interference between the first and second sample dispensing mechanisms when they delivery biochemical and electrolyte samples, respectively.

In some embodiments, the second rotation center 413 is located between the first rotation center 421 and the third rotation center 414. In a specific embodiment, the sample needle of the first sample delivery arm 411 is used to transfer the samples from the sample containers at the shared sample aspiration position 211 to the reaction vessels carried by the inner reaction ring 12, and the sample needle of the second sample delivery arm 412 is used to transfer the samples from the sample containers at the shared sample aspiration position 211 to the reaction vessels carried by the outer reaction ring 11.

In one embodiment, the samples located on the first track 21 and the samples located on the second track 22 enter the sample analyzer 100 through the first track 21.

In some embodiments, the sample analyzer 100 also includes a sample rack transfer mechanism 23. The second sample rack is transferred from the first track 21 to the second track 22 by the sample rack transfer mechanism 23, enabling the second sample dispensing mechanism to aspirate sample on the second track 22. This arrangement avoids the second sample dispensing mechanism sampling on the first track 21, thereby achieving independent aspiration of the first and second sample dispensing mechanisms. In this way, the sample analyzer 100 can efficiently perform biochemical sampling while simultaneously handling electrolyte analyzing sampling without slowing down.

In some embodiments, the sample analyzer 100 further includes a third track 24, which is used for recycle the sample rack. After respective sampling tasks of the first and second sample racks are completed respectively, the sample rack transfer mechanism 23 transfers the first and second sample racks to the third track 24, and the first and second sample racks exit the sample analyzer 100 via the third track 24.

In one embodiment, the first track 21, the second track 22, and the third track 24 are parallel to each other. The sample rack transfer mechanism 23 can also be used to transfer the first sample rack on the first track 21 or the second sample rack on the second track 22 to the third track 24.

Specifically, the sample rack transfer mechanism 23 can perform sample rack transfer between the first track 21 and the second track 22, between the first track 21 and the third track 24, and between the second track 22 and the third track 24.

In one embodiment, the sample rack transfer mechanism 23 includes a transfer track 231 and a first driving device 232. The first driving device 232 is used to drive the transfer track 231 to connect with the first discharge position 213 of the first track 21, so as to transfer the first sample rack on the first track 21 or the second sample rack to the transfer track 231.

The first driving device 232 is also used to drive the transfer track 231 to connect with the access position 221 of the second track 22, so as to transfer the second sample rack on the transfer track 231 to the second track 22, or to transfer the second sample rack from the second track 22 to the transfer track 231.

The first driving device 232 is also used to drive the transfer track 231 to connect with the third feed position 241 of the third track 24, so as to transfer the first or second sample rack on the transfer track 231 to the third track 24 and make the first or second sample rack exit the sample analyzer 100 via the third track 24.

In another embodiment, the sample rack transfer mechanism 23 includes a second driving device and a gripper. Under the drive of the second driving device, the gripper can transfer the second sample rack on the first track 21 to the second track 22, allowing the second sample dispensing mechanism to aspirate the sample needed for electrolyte analyzing from the second sample rack.

Under the drive of the second driving device, the gripper can also transfer the first sample rack on the first track 21 to the third track 24, and transfer the second sample rack on the second track 22 to the third track 24, allowing the first or second sample rack to exit the sample analyzer 100 via the third track 24.

In some embodiments that are more specific, the second driving device can be a three-dimensional moving module or a robotic arm, which can drive the gripper to transfer sample racks among the first track 21, the second track 22, and the third track 24.

Furthermore, in one embodiment, the first track 21 is used to transport the first and second sample racks. The first sample rack carries sample containers containing samples that need biochemical analyzing.

After the sample aspiration required for biochemical analysis in the sample containers of the first sample rack has been completed by the first sample dispensing mechanism, the first sample rack moves along the first track 21 towards the sample rack transfer mechanism 23, and is transferred to the transfer track 231. Then, the first driving device 232 drives the transfer track 231 to connect with the third feed position 241 of the third track 24, transferring the first sample rack to the third track 24. Finally, the first sample rack moves along the third track 24 and exits the sample analyzer 100.

Alternatively, after the sample aspiration required for biochemical analysis in the sample containers of the first sample rack has been completed by means of the first sample dispensing mechanism, the second driving device drives the gripper to grip the first sample rack and transfer the first sample rack to the third track 24.

In some embodiments, the second sample rack is transferred from the first track 21 to the second track 22 and carries samples that require electrolyte analyzing.

In one embodiment, the sample containers on the second sample rack contain samples that need electrolyte analyzing. When the samples contained in the sample containers carried by the second sample rack pass through the shared sample aspiration position 211 on the first track 21, they are sampled either by the sample needle of the first sampling arm 411 or by the sample needle of the second sampling arm 412. Subsequently, the samples move to the second track 22, where they are aspirated by the sample needle of the second sampling mechanism. In this way, the sample containers carried by the second sample rack may supply samples for both the first sampling mechanism and the second sampling mechanism along their movement path. As a result, it improves the efficiency of both the sample transportation and sample aspiration.

Specifically, when the second sample rack enters the sample analyzer 100 from the first track 21, the second sample rack moves along the first track 21 to the first discharge position 213 of the first track 21, and then moves from the first discharge position 213 to the transfer track 231.

Afterward, the first driving device 232 drives the transfer track 231, carrying the second sample rack, to connect with the access position 221 of the second track 22. After the connection, the second sample rack is transferred to the second track 22, allowing the second sample dispensing mechanism to take the samples needed for electrolyte analyzing.

After the samples for electrolyte analyzing have been completely aspirated by means of the second sample dispensing mechanism from the second sample rack, the second sample rack moves to the access position 221 of the second track 22 and is transferred to the transfer track 231.

Subsequently, the first driving device 232 drives the transfer track 231 to connect with the third feed position 241 of the third track 24, and transfers the second sample rack to the third track 24. Finally, the second sample rack exits the sample analyzer 100 via the third track 24.

Alternatively, when the second sample rack enters the sample analyzer 100 from the first track 21, the second driving device drives the gripper to grip the second sample rack and transfer the second sample rack to the second track 22. Subsequently, the second sample dispensing mechanism takes the sample required for electrolyte analyzing from the sample containers carried by the second sample rack.

After the samples for electrolyte analyzing has been completely aspirated by means of the second sample dispensing mechanism from the second sample rack, the second driving device further drives the gripper to grip the second sample rack and transfer the second sample rack to the third track 24, allowing the second sample rack to exit the sample analyzer 100 via the third track 24.

In another embodiment, the sample containers carried by the second sample rack contain both samples for electrolyte analyzing and samples for biochemical analyzing.

Specifically, when the samples required for biochemical analyzing are completely aspirated by means of the first sample dispensing mechanism from the sample containers on the second sample rack, the second sample rack moves along the first track 21 towards the sample rack transfer mechanism 23 and is transferred to the transfer track 231.

Subsequently, the first driving device 232 drives the transfer track 231 to connect with the access position 221 of the second track 22, and after the connection, the second sample rack is transferred to the second track 22, allowing the second sample dispensing mechanism to aspirate the samples needed for electrolyte analyzing.

When the samples for electrolyte analyzing are completely aspirated by means of the second sample dispensing mechanism from the second sample rack, the second sample rack moves to the access position 221 of the second track 22 and is transferred to the transfer track 231.

Subsequently, the first driving device 232 drives the transfer track 231 to connect with the third feed position 241 of the third track 24, transferring the second sample rack to the third track 24. Finally, the second sample rack exits the sample analyzer 100 via the third track 24.

Alternatively, when the second sample rack enters the sample analyzer 100 from the first track 21, the first sample dispensing mechanism collects the sample needed for biochemical analyzing from the sample containers carried by the second sample rack on the first track 21. After the samples have been completely aspirated by means of the first sample dispensing mechanism, the second driving device drives the gripper to grip the second sample rack and transfer the second sample rack to the second track 22.

After the second sample rack is transferred to the second track 22, the second sample dispensing mechanism collects the sample needed for electrolyte analyzing from the sample containers carried by the second sample rack.

After the samples have been completely aspirated by means of the second sample dispensing mechanism, the second driving device drives the gripper to grip the second sample rack and transfer the second sample rack to the third track 24, and then the second sample rack exits the sample analyzer 100 via the third track 24.

Furthermore, the sample analyzer 100 also includes a fourth track 25, which is at least used to transport a third sample rack. The sample containers carried by the third sample rack contain emergency samples.

Specifically, the fourth track 25 is parallel to the first track 21, the second track 22, and the third track 24, and the transfer track 231 can connect with the fourth track 25. Alternatively, the second driving device can drive the gripper to grip the third sample rack on the fourth track 25.

The first sample dispensing mechanism may further collect the emergency samples needed for biochemical analysis from the third sample rack on the fourth track 25.

Specifically, the first sample delivery arm 411 rotates around the second rotation center 413, such that the sample needle of the first sample delivery arm 411 intersects with the fourth track 25 at an intersection that serves as a first emergency sample aspiration position 251. The second sample delivery arm 412 rotates around the third rotation center 414, such that the sample needle of the second sample delivery arm 412 intersects with the fourth track 25 at another intersection that serves as a second emergency sample aspiration position 252.

The third sample rack moves along the fourth track 25, allowing the multiple sample containers carried on the third sample rack to sequentially pass through the second emergency sample aspiration position 252 and the first emergency sample aspiration position 251.

In case of the single-ring structure of the reaction wheel 10, the first sample dispensing mechanism samples from the sample containers located at the first emergency sample aspiration position 251 and transfers the samples to the reaction vessels located at the sample dispensing position.

In case of the double-ring structure of the reaction wheel 10, the sample needle of the first sample delivery arm 411 samples from the sample containers at the first emergency sample aspiration position 251 and transfers the samples to the reaction vessels located at the inner sample dispensing position 121. The sample needle of the second sample delivery arm 412 samples from the sample containers at the second emergency sample aspiration position 252 and transfers the samples to the reaction vessels located at the outer sample dispensing position 111.

Furthermore, a first cleaning position 415 is provided beneath the movement trajectory of the sample needle of the first sample delivery arm 411. The first cleaning position 415 corresponds to a point of the movement trajectory of the sample needle between the shared sample aspiration position 211 and the inner sample dispensing position 121, and is at the side away from the second rotation center 413.

A second cleaning position 416 is provided beneath the movement trajectory of the sample needle of the second sample delivery arm 412. The second cleaning position 416 is extends along the movement trajectory of the sample needle between the shared sample aspiration position 211 and the outer sample dispensing position 111, and is also at the side away from the second rotation center 413.

In one embodiment, the second sample rack, which is used only for deliver samples for biochemical analyzing, can also enter the sample analyzer 100 via the fourth track 25. The second sample rack can then be transferred from the fourth track 25 to the second track 22 by the sample rack transfer mechanism 23.

Specifically, in case that the samples in the sample containers carried by the first sample rack or the second sample rack on the first track 21 are being sampled by the first sample dispensing mechanism, and there are no emergency samples for biochemical analysis on the fourth track 25, the second sample rack, which carries samples only for electrolyte analyzing, can enter the sample analyzer 100 via the fourth track 25. The second sample rack can then be transferred to the second track 22 by the sample rack transfer mechanism 23, enabling the second sample dispensing mechanism to perform sampling.

Furthermore, the sample analyzer 100 also includes a reagent container storage mechanism and a reagent injection mechanism. The reagent container storage mechanism includes the first and second reagent container storage mechanisms. The reagent injection mechanism includes the first reagent injection mechanism 62 and the second reagent injection mechanism 63.

The first reagent container storage mechanism is located on the outside of the reaction wheel 10 and can carry multiple reagent containers. The first reagent container storage mechanism can rotate to move multiple reagent containers along the first rotation path.

The first reagent container storage mechanism along the first rotation path is configured with multiple first carrying positions, with each first carrying position capable of carrying a reagent container.

The first reagent container storage mechanism includes the first reagent aspirating position 51 and the second reagent aspirating position 52. The first reagent container storage mechanism rotates around the first rotation center 535 to move the multiple first carrying positions to sequentially pass through the first reagent aspirating position 51 and the second reagent aspirating position 52.

The reaction wheel 10 can carry multiple reaction vessels, and the reaction wheel 10 can rotate to move multiple reaction vessels along the third rotation path.

Specifically, in case that the reaction wheel 10 is a single-ring structure, the reaction wheel 10 is configured with multiple third carrying positions along the third rotation path, and each third carrying position can hold a reaction vessel.

The reaction wheel 10 includes a first reagent dispensing position 13 and a second reagent dispensing position 14. The reaction wheel 10 rotates around the third rotation center 15, causing the multiple third carrying positions to sequentially pass through the first reagent dispensing position 13 and the second reagent dispensing position 14.

The first reagent injection mechanism 62 includes a first reagent needle 61 and a second reagent needle which operate independently.

The first reagent needle 61 is used to withdraw reagents from the reagent container located at one of the first reagent aspirating position 51 and the second reagent aspirating position 52, then transfer the reagent along the first straight line 551 and dispense the reagents into the reaction vessel located at one of the first reagent dispensing position 13 and the second reagent dispensing position 14.

The second reagent needle is used to withdraw reagents from the reagent container located at the other of the first reagent aspirating position 51 and the second reagent aspirating position 52, then transfer the reagent along the second straight line 552 and dispense the reagents into the reaction vessel located at the other of the first reagent dispensing position 13 and the second reagent dispensing position 14.

The reaction wheel 10 rotates to move the two target reaction vessels to the first reagent dispensing position 13 and the second reagent dispensing position 14, and the first reagent container storage mechanism rotates to move the two target reagent containers to the first reagent aspirating position 51 and the second reagent aspirating position 52.

Subsequently, the first reagent needle 61 will withdraw reagents from the target reagent containers located at one of the first reagent aspirating position 51 and the second reagent aspirating position 52, and the reagents are transferred along the first straight line 551 and are dispensed into the target reaction vessel located at one of the first reagent dispensing position 13 and the second reagent dispensing position 14.

The second reagent needle will withdraw reagents from the target reagent containers located at the other of the first reagent aspirating position 51 and the second reagent aspirating position 52, and the reagents are transferred along the second straight line 552 and are dispensed into the target reaction vessel located at the other of the first reagent dispensing position 13 and the second reagent dispensing position 14.

Compared to the reagent dispensing methods in conventional sample analyzers 100, in this embodiment, the first reagent needle 61 operates independently along the first straight line 551 for reagent transfer, and the second reagent needle operates independently along the second straight line 552 for reagent transfer. This significantly improves the efficiency of dispensing reagents into reaction vessels. It avoids the need for the first and second reagent needles to sequentially perform reagent withdrawal and dispensing, as well as the rotation required for reagent transfer and resetting, thereby effectively saving the waiting time for the reaction wheel 10 while the first reagent needle 61 and second reagent needle are dispensing reagents. It also reduces the waiting time for the first reagent container storage mechanism while the first reagent needle 61 and second reagent needle are withdrawing reagents, as well as the reagent transfer time and the resetting time for both probes. Moreover, the independently operating first reagent needle 61 and second reagent needle can be controlled according to the needs to transfer reagents with only one probe at a time.

In one embodiment, the first reagent container storage mechanism has a single-ring structure, and the multiple first carrying positions are used to transfer reaction vessels along the first rotation path around the first rotation center 535.

In another embodiment, the first reagent container storage mechanism has a double-ring structure, including a first inner storage ring of the reagent container 531 and a first outer storage ring of the reagent container 533. The first inner storage ring of the reagent container 531 is positioned inside the first outer storage ring of the reagent container 533.

The first inner storage ring of the reagent container 531 is configured with multiple first inner carrying positions 532 along the first inner rotation path, and each first inner carrying position 532 can hold a reagent container.

The first outer storage ring of the reagent container 533 is configured with multiple first outer carrying positions 534 along the first outer rotation path, and each first outer carrying position 534 can hold a reagent container.

Furthermore, in a more specific embodiment, the first reagent aspirating position 51 is located on the first inner rotation path of the first inner storage ring of the reagent container 531, and the multiple first inner carrying positions 532 move along the first inner rotation path, passing sequentially through the first reagent aspirating position 51. The second reagent aspirating position 52 is located on the first outer rotation path of the first outer storage ring of the reagent container 533, and the multiple first outer carrying positions 534 move along the first outer rotation path, passing sequentially through the second reagent aspirating position 52.

In another more specific embodiment, the first reagent aspirating position 51 is located on the first outer rotation path of the first outer storage ring of the reagent container 533, and the multiple first outer carrying positions 534 move along the first outer rotation path, passing sequentially through the first reagent aspirating position 51. The second reagent aspirating position 52 is located on the first inner rotation path of the first inner storage ring of the reagent container 531, and the multiple first inner carrying positions 532 move along the first inner rotation path, passing sequentially through the second reagent aspirating position 52.

In one embodiment, the first inner storage ring of the reagent container 531 and the first outer storage ring of the reagent container 533 rotate synchronously.

In another embodiment, the first inner storage ring of the reagent container 531 can rotate independently relative to the first outer storage ring of the reagent container 533. That is, when the first inner storage ring of the reagent container 531 rotates, the first outer storage ring of the reagent container 533 can remain stationary or rotate at the same or a different speed. Alternatively, when the first outer storage ring of the reagent container 533 rotates, the first inner storage ring of the reagent container 531 can remain stationary or rotate at the same or a different speed.

In some embodiments, the independently rotating first inner storage ring of the reagent container 531 can control the target reagent container to directly move to the first reagent aspirating position 51 or the second reagent aspirating position 52 corresponding to the first reagent needle 61, based on the type of the reagent to be drawn in the reagent container held at the first inner carrying position 532.

The independently rotating first outer storage ring of the reagent container 533 can control the target reagent container to directly move to the second reagent aspirating position 52 or the first reagent aspirating position 51 corresponding to the second reagent needle, based on the type of the reagent to be drawn in the reagent container held at the first outer carrying position 534.

Compared to the first inner storage ring of the reagent container 531 and the first outer storage ring of the reagent container 533 which rotate synchronously, the independently rotating of the first inner storage ring of the reagent container 531 and the first outer storage ring of the reagent container 533 make it easier for the first reagent needle 61 and the second reagent needle to perform reagent intake. In other words, this prevents the situation where, when the first reagent needle 61 draws reagent from the target reagent container, the target reagent container corresponding to the second reagent needle is unable to simultaneously reach the second reagent aspirating position 52, which leads to the requirement of the first reagent needle 61 drawing the reagent from the target reagent container, followed by the reaction wheel 10 rotating again to move the target reagent container corresponding to the second reagent needle to the second reagent aspirating position 52, where the second reagent needle would then perform the intake. As a result, during this process, the target reagent container corresponding to the second reagent needle has to stop twice during the rotation of the reaction wheel 10 to reach the second reagent aspirating position 52.

Further, in a more specific embodiment, the first reagent dispensing position 13 is located on the third inner rotation path of the inner reaction ring 12. The multiple third inner carrying positions 122 move along the third inner rotation path and pass sequentially through the first reagent dispensing position 13.

The second reagent dispensing position 14 is located on the third outer rotation path of the outer reaction ring 11. The multiple third outer carrying positions 112 move along the third outer rotation path and pass sequentially through the second reagent dispensing position 14.

In another more specific embodiment, the first reagent dispensing position 13 is located on the third outer rotation path of the outer reaction ring 11. The multiple third outer carrying positions 112 move along the third outer rotation path and pass sequentially through the first reagent dispensing position 13.

The second reagent dispensing position 14 is located on the third inner rotation path of the inner reaction ring 12. The multiple third inner carrying positions 122 move along the third inner rotation path and pass sequentially through the second reagent dispensing position 14.

In one embodiment, the inner reaction ring 12 and the outer reaction ring 11 rotate synchronously.

In another embodiment, the inner reaction ring 12 can rotate independently relative to the outer reaction ring 11. That is, when the inner reaction ring 12 rotates, the outer reaction ring 11 can remain stationary or rotate at the same or a different speed. Alternatively, when the outer reaction ring 11 rotates, the inner reaction ring 12 can remain stationary or rotate at the same or a different speed.

In some embodiments, the independently rotating inner reaction ring 12 can control the target reaction vessels to directly move to the first reagent dispensing position 13 or the second reagent dispensing position 14 corresponding to the first reagent needle 61, based on the type of the reagent to be drawn in the reaction vessels held at the third inner carrying positions 122.

The independently rotating outer reaction ring 11 can control the target reaction vessels to directly move to the second reagent dispensing position 14 or the first reagent dispensing position 13 corresponding to the second reagent needle, based on the type of the reagent to be drawn in the reaction vessels held at the third outer carrying position 112.

Compared to the inner reaction ring 12 and the outer reaction ring 11 that rotate synchronously, the independently rotating of the inner reaction ring 12 and the outer reaction ring 11 make it easier for the first reagent needle 61 and second reagent needle to draw reagents.

In some embodiments, by providing the inner reaction ring 12 and outer reaction ring 11 that can rotate independently, as well as the first inner storage ring of the reagent container 531 and the first outer storage ring of the reagent container 533 that can rotate independently, the first reagent needle 61 operates independently to draw reagents from the reagent containers held by the first outer storage ring of the reagent container 533 to the reaction vessels held by the inner reaction ring 12 or outer reaction ring 11, and the second reagent needle operates independently to draw reagents from the reagent containers held by the first inner storage ring of the reagent container 531 to the outer reaction ring 11 or inner reaction ring 12. In this way, the sample analyzer 100 can integrate two independent systems for supplying, dispensing, and detecting reagents, significantly improving the analyzing efficiency of the sample analyzer 100.

Further, in one embodiment, a first center connection is formed between the first rotation center 535 and the third rotation center 15. The first straight line 551 is parallel to the first center connection; or the first straight line 551 directly intersects with the first center connection; or the first straight line 551 intersects with an extended line of the first center connection. Similarly, the second straight line 552 is parallel to the first center connection; alternatively, the second straight line 552 directly intersects with the first center connection; or the second straight line 552 intersects with the extended line of the first center connection.

In some embodiments, the first straight line 551 and the second straight line 552 are parallel to the first center connection, and the distance between the first straight line 551 and the first center connection is equal to the distance between the second straight line 552 and the first center connection.

In some embodiments, the time it takes for the first reagent needle 61 to move from the first reagent aspirating position 51 to the first reagent dispensing position 13 along the first straight line 551 is the same as the time it takes for the second reagent needle to move from the second reagent aspirating position 52 to the second reagent dispensing position 14 along the second straight line 552.

In one embodiment, the first reagent needle 61 and the second reagent needle are respectively provided on a support and arranged spaced apart with a certain distance.

In some embodiments, the first reagent needle 61 is vertically moved via a lifting drive device to allow the first reagent needle 61 to move between one of the first reagent aspirating position 51 and the second reagent aspirating position 52 and one of the first reagent dispensing position 13 and the second reagent dispensing position 14.

Further, the first reagent needle 61 is also moved horizontally along the first straight line 551 via a horizontal drive device, enabling the first reagent needle 61 to move between the first reagent aspirating position 51 and either the first reagent dispensing position 13 or the second reagent dispensing position 14, or between the second reagent aspirating position 52 and either the second reagent dispensing position 14 or the first reagent dispensing position 13.

The second reagent needle moves vertically through another lifting drive device, allowing the second reagent needle to move between one of the first reagent aspirating position 51 and the second reagent aspirating position 52 and one of the first reagent dispensing position 13 and the second reagent dispensing position 14.

Furthermore, the second reagent needle also moves horizontally along the second straight line 552 through another horizontal drive device, allowing the second reagent needle to move between the first reagent aspirating position 51 and either the first reagent dispensing position 13 or the second reagent dispensing position 14, or between the second reagent aspirating position 52 and either the second reagent dispensing position 14 or the first reagent dispensing position 13.

In another embodiment, the first reagent needle 61 and the second reagent needle are positioned on the same support. The first reagent needle 61 and the second reagent needle move vertically via separate lifting drive devices and move horizontally along their respective first straight line 551 and second straight line 552 via respective horizontal drive devices.

Furthermore, the reaction wheel 10 also includes a third reagent dispensing position 16 and a fourth reagent dispensing position 17. The reaction wheel 10, rotating around the third rotation center 15, can drive multiple third carrying positions to sequentially pass through the third reagent dispensing position 16 and the fourth reagent dispensing position 17.

The second reagent injection mechanism 63 is used to transfer reagents from the reagent containers held by the second reagent container storage mechanism to the reaction vessels carried by the reaction wheel 10.

The second reagent container storage mechanism is located on the outer side of the reaction wheel 10 and can carry multiple reagent containers. The second reagent container storage mechanism can rotate to move multiple reagent containers along the second rotation path.

The second reagent container storage mechanism is provided with multiple second carrying positions along the second rotation path, each of the second carrying positions can hold one reagent container.

The second reagent container storage mechanism includes a third reagent aspirating position 57 and a fourth reagent aspirating position 58. The second reagent container storage mechanism, rotating around the second rotation center 545, can drive multiple second carrying positions to sequentially pass through the third reagent aspirating position 57 and the fourth reagent aspirating position 58.

The second reagent injection mechanism 63 includes a third reagent needle and a fourth reagent needle which operate independently.

The third reagent needle is used to extract reagents from the reagent container located at one of the third reagent aspirating position 57 and the fourth reagent aspirating position 58, transfer the reagent along the third straight line 561, and drain the reagent into a reaction vessel located at one of the third reagent dispensing position 16 and the fourth reagent dispensing position 17.

The fourth reagent needle is used to extract reagents from the reagent container located at the other of the third reagent aspirating position 57 and the fourth reagent aspirating position 58, transfer the reagent along the fourth straight line 562 and drain the reagent into the reaction vessel located at the other of the third reagent dispensing position 16 and the fourth reagent dispensing position 17.

The reaction wheel 10 rotates to move the two target reaction vessels respectively to the third reagent dispensing position 16 and the fourth reagent dispensing position 17, and the second reagent container storage mechanism rotates to move the two target reagent containers respectively to the third reagent aspirating position 57 and the fourth reagent aspirating position 58.

The third reagent needle will extract reagents from the target reagent container located at one of the third reagent aspirating position 57 and the fourth reagent aspirating position 58, and transfer the extracted reagent along the third straight line 561 to drain the reagent into the target reaction vessel located at one of the third reagent dispensing position 16 and the fourth reagent dispensing position 17.

The fourth reagent needle will extract reagents from the target reagent container located at the other of the third reagent aspirating position 57 and the fourth reagent aspirating position 58, and transfer the extracted reagent along the fourth straight line 562 to dispense the reagent into the target reaction vessel located at the other of the third reagent dispensing position 16 and the fourth reagent dispensing position 17.

In one embodiment, the second reagent container storage mechanism is a single-ring structure, and the multiple second carrying positions rotate along the second rotation path around the second rotation center 545 to transfer reaction vessels.

In another embodiment, the second reagent container storage mechanism is a dual-ring structure, including a second inner storage ring of the reagent container 541 and a second outer storage ring of the reagent container 543, and the second inner storage ring of the reagent container 541 is located inside the second outer storage ring of the reagent container 543.

The second inner storage ring of the reagent container 541 is provided with multiple second inner carrying positions 542 along the second inner rotation path, and each of the second inner carrying positions 542 can carry one reagent container.

The second outer storage ring of the reagent container 543 is provided with multiple second outer carrying positions 544 along the second outer rotation path, and each of the second outer carrying positions 544 can carry one reagent container.

Furthermore, in a more specific embodiment, the third reagent aspirating position 57 is located on the second inner rotation path of the second inner storage ring of the reagent container 541. Multiple second inner carrying positions 542 move along the second inner rotation path and pass through the third reagent aspirating position 57 sequentially.

The fourth reagent aspirating position 58 is located on the second outer rotation path of the second outer storage ring of the reagent container 543. Multiple second outer carrying positions 544 move along the second outer rotation path and pass through the fourth reagent aspirating position 58 sequentially.

In another more specific embodiment, the third reagent aspirating position 57 is located on the second outer rotation path of the second outer storage ring of the reagent container 543. Multiple second outer carrying positions 544 move along the first outer rotation path and pass through the third reagent aspirating position 57 sequentially.

The fourth reagent aspirating position 58 is located on the second inner rotation path of the second inner storage ring of the reagent container 541. Multiple second inner carrying positions 542 move along the second inner rotation path and pass through the fourth reagent aspirating position 58 sequentially.

In one embodiment, the second inner storage ring of the reagent container 541 and the second outer storage ring of the reagent container 543 rotate synchronously.

In another embodiment, the second inner storage ring of the reagent container 541 can rotate independently relative to the second outer storage ring of the reagent container 543. That is, when the second inner storage ring of the reagent container 541 rotates, the second outer storage ring of the reagent container 543 can remain stationary or rotate at the same speed or a different speed; or, when the second outer storage ring of the reagent container 543 rotates, the second inner storage ring of the reagent container 541 can remain stationary or rotate at the same speed or a different speed.

Furthermore, the independently rotatable second inner storage ring of the reagent container 541 can control the target reagent container to move directly to the third reagent aspirating position 57 or the fourth reagent aspirating position 58 corresponding to the third reagent needle based on the type of the reagent to be drawn in the reagent container carried on the second inner carrying position 542.

The independently rotatable second outer storage ring of the reagent container 543 can control the target reagent container to move directly to the third reagent aspirating position 57 or the fourth reagent aspirating position 58 corresponding to the fourth reagent needle based on the type of the reagent to be drawn in the reagent container carried on the second outer carrying position 544.

Furthermore, in one embodiment, a second center connection line is formed between the second rotation center 545 and the third rotation center 15. The third straight line 561 is parallel to the second center connection line; or the third straight line 561 directly intersects with the second center connection line; or the third straight line 561 intersects with an extension line of the second center connection line. The fourth straight line 562 is parallel to the second center connection line; or the fourth straight line 562 directly intersects with the second center connection line; or the fourth straight line 562 intersects with an extension line of the second center connection line.

Preferably, the third straight line 561 and the fourth straight line 562 are both parallel to the second center connection line, and the distance from the third straight line 561 to the second center connection line is equal to the distance from the fourth straight line 562 to the second center connection line.

Furthermore, the reaction wheel 10 has a horizontal symmetry line 102 and a vertical symmetry line 104 both passing through the third rotation center 15. The horizontal symmetry line 102 and the vertical symmetry line 104 divide the reaction wheel 10 into the first quadrant, second quadrant, third quadrant, and fourth quadrant.

In one embodiment, the first rotation center 535 and the second rotation center 545 are located on opposite sides of the vertical symmetry line 104 and on the same side of the horizontal symmetry line 102, so that the first reagent container storage mechanism and the second reagent container storage mechanism are separately located in the first and second quadrants at an outer side of the reaction wheel 10, or separately located in the third and fourth quadrants and at an outer side of the reaction wheel 10.

In another embodiment, the first rotation center 535 and the second rotation center 545 are located on opposite sides of the horizontal symmetry line 102 and on the same side of the vertical symmetry line 104, so that the first reagent container storage mechanism and the second reagent container storage mechanism are separately located in the first and fourth quadrants at an outer side of the reaction wheel 10, or separately located in the second and third quadrants and at an outer side of the reaction wheel 10.

Specifically, the reaction wheel 10, the first reagent container storage mechanism, and the second reagent container storage mechanism are all arranged on the support frame, and the first reagent container storage mechanism and the second reagent container storage mechanism are located on the same side of the support frame to facilitate the loading and unloading of reagent containers in both mechanisms.

Furthermore, a central line 103 is formed by connecting the first rotation center 535 of the first reagent container storage mechanism and the second rotation center 545 of the second reagent container storage mechanism.

The point of the vertical symmetry line 104 intersecting the center line 103 is located at the midpoint of the central line 103.

Specifically, the straight-line distance from the first rotation center 535 to the third rotation center 15 is equal to the straight-line distance from the second rotation center 545 to the third rotation center 15, so that a triangle formed by connecting lines between every two of the first rotation center 535, the second rotation center 545, and the third rotation center 15 is an isosceles triangle, and the first reagent container storage mechanism and the second reagent container storage mechanism are symmetrically distributed on both sides of the reaction wheel 10 along the vertical symmetry line 104.

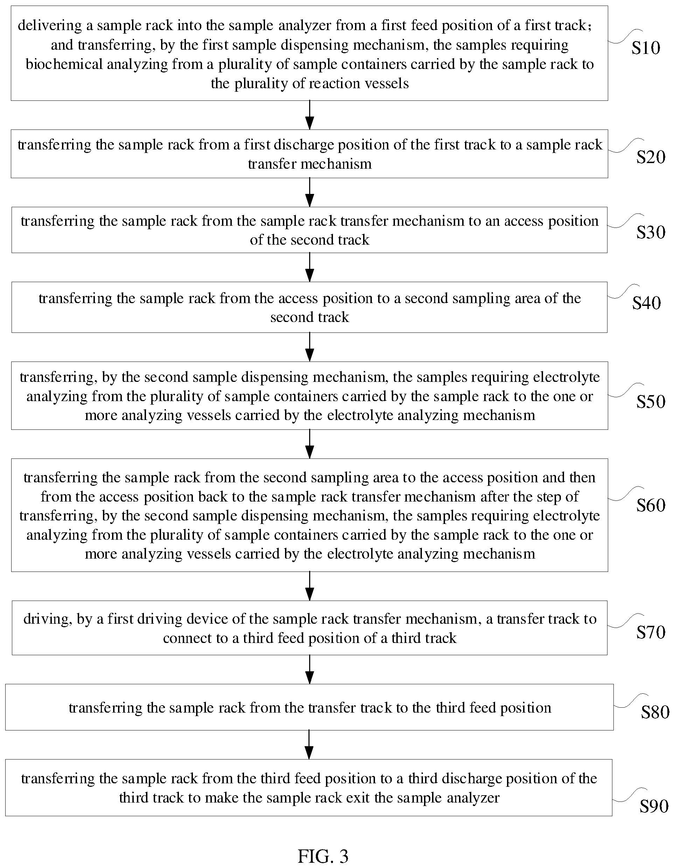

Referring to Figure 3, on the other aspect, the present invention also provides a sample transportation method for a sample analyzer 100, which includes the following steps:

Step S10: delivering a sample rack into the sample analyzer 100 from a first feed position 212 of a first track 21; and transferring, by the first sample dispensing mechanism, the samples requiring biochemical analyzing from a plurality of sample containers carried by the sample rack to the plurality of reaction vessels.

In this step, the first track 21 is located on the outer side of the reaction wheel 10, which is placed on the support frame and carries reaction vessels used to contain samples that need biochemical analyzing.

The sample rack carries sample containers, and the samples in the sample containers are in need of biochemical and/or electrolyte analyzing.

The first track 21 is arranged on the outer side of the reaction wheel 10 and is used to transport the sample rack. The first track 21 includes a first feed position 212 and a first discharge position 213. The first feed position 212 is the entry end of the sample rack into the first track 21, and the first discharge position 213 is the exit end of the sample rack leaving the first track 21.

Step S20: transferring the sample rack from a first discharge position 213 of the first track 21 to a sample rack transfer mechanism 23.

In this step, the sample rack transfer mechanism 23 includes a transfer track 231 and a first driving device 232. The first driving device 232 is used to drive the transfer track 231 to connect with the first discharge position 213 of the first track 21, so that the first sample rack or the second sample rack on the first track 21 can be transferred to the transfer track 231.

Specifically, the sample rack is conveyed through the first track 21, and when the transfer track 231 connects with the first discharge position 213 of the first track 21, the first track 21 conveys the sample rack to the transfer track 231.

Step S30: transferring the sample rack from the sample rack transfer mechanism 23 to an access position 221 of a second track 22.

In this step, after the sample rack is transferred to the transfer track 231, the first driving device 232 drives the transfer track 231 carrying the sample rack to move and connect with the access position 221 of the second track 22. Subsequently, the sample rack is conveyed through the transfer track 231 to the access position 221 of the second track 22.

Step S40: transferring the sample rack from the access position 221 to a second sampling area of the second track 22.

In this step, after the sample rack reaches the access position 221 of the second track 22, the sample rack is conveyed through the second track 22 until the sample rack moves to the second sampling area of the second track 22.

Step S50: transferring, by a second sample dispensing mechanism, the samples requiring electrolyte analyzing from the plurality of sample containers carried by the sample rack to the one or more analyzing vessels carried by the electrolyte analyzing mechanism 30.

After the sample rack moves to the second sampling area, the second sample dispensing mechanism takes the required sample for electrolyte analyzing from the sample container carried by the sample rack in the second sampling area and adds the taken sample to the analyzing vessel located at the first or second sample injection position 31 or 32.

After the sample for electrolyte analyzing is added to the analyzing vessel, the electrolyte analyzing mechanism 30 will perform electrolyte analyzing on the sample.

Step S60: transferring the sample rack from the second sampling area to the access position 221 and then from the access position 221 back to the sample rack transfer mechanism 23 after the step S50.

In this step, after the samples have been transferred by the second sample dispensing mechanism from the sample container carried by the sample rack, the sample rack moves towards the access position 221 until the sample rack reaches the access position 221.

Before or after the sample rack reaches the access position 221, the first driving device 232 drives the transfer track 231 to connect with the access position 221 of the second track 22.

After the transfer track 231 connects with the access position 221, the second track 22 conveys the sample rack onto the transfer track 231, which is then moved by the second driving device to exit the sample analyzer 100.

Further, during the process in which the second sample dispensing mechanism transfers the sample from the sample container carried by the sample rack to the analyzing vessel, the sample rack remains stationary on the second track 22. The second sample dispensing mechanism can move to different sample containers carried by the sample rack to aspirate the sample.

Specifically, the sample needle of the second sample dispensing mechanism can move to different sample containers carried by the sample rack, which is stationary on the second track 22, to aspirate the sample.

In one embodiment, the second sample dispensing mechanism includes a folding arm and a sample needle. The folding arm can drive the sample needle to move to different sample containers carried by the sample rack to aspirate the sample.

Specifically, the folding arm includes at least a first folding sub-arm 422 and a second folding sub-arm 423. One end of the first folding sub-arm 422 is arranged rotatably about the first rotation center 421, and the other end of the first folding sub-arm 422 is connected to one end of the second folding sub-arm 423. The second folding sub-arm 423 can rotate relative to the first folding sub-arm 422, and the other end of the second folding sub-arm 423 is connected to the sample needle.

By providing the sample needle on the folding arm, the sample needle can perform sampling in the second sampling area on the second track 22. The second sampling area can accommodate at least one sample rack, allowing the sample needle to sample from any sample container in the sample rack that is stationary in the second sampling area.

In another embodiment, the second sample dispensing mechanism includes a telescopic arm and a sample needle. The telescopic arm can drive the sample needle to move to different sample containers carried by the sample rack to aspirate the sample.

Specifically, the telescopic arm includes at least a first telescopic sub-arm and a second telescopic sub-arm. One end of the first telescopic sub-arm is arranged rotatably about the first rotation center 421, and the other end of the first telescopic sub-arm is connected to one end of the second telescopic sub-arm. The second telescopic sub-arm is telescopic relative to the first telescopic sub-arm, and the other end of the second telescopic sub-arm is connected to the sample needle.

By providing the sample needle on the telescopic arm, the sample needle can perform sampling in the second sampling area on the second track 22. The second sampling area can accommodate at least one sample rack, allowing the sample needle to sample from any sample container in the sample rack that is stationary in the second sampling area.

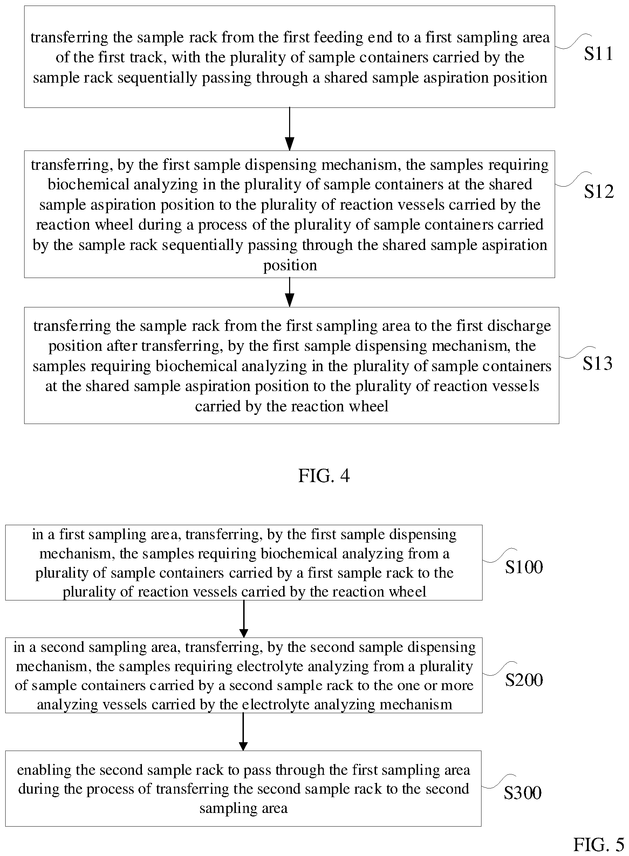

As shown in FIG. 4, the sample rack is delivered into the sample analyzer 100 from the first feed position 212 of the first track 21, and the method further includes:

Step S11: transferring the sample rack from the first feeding end 212 to a first sampling area of the first track 21, with the plurality of sample containers carried by the sample rack sequentially passing through a shared sample aspiration position 211.

In this step, the first sampling area is located between the first feed position 212 and the first discharge position 213 of the first track 21. The sample rack moves on the first track 21, allowing each sample container in the sample rack to sequentially reach the shared sample aspiration position 211.

Step S12: transferring, by the first sample dispensing mechanism, the samples requiring biochemical analyzing in the plurality of sample containers at the shared sample aspiration position 211 to the plurality of reaction vessels carried by the reaction wheel 10 during a process of the plurality of sample containers carried by the sample rack sequentially passing through the shared sample aspiration position 211.

Specifically, the sample added to the reaction vessel carried by the reaction wheel 10 is used for biochemical analyzing.

Step S13: transferring the sample rack from the first sampling area to the first discharge position 213 after the step S12.

In this step, after the samples have been completely aspirated by means of the first sample convey mechanism from the sample container carried by the sample rack in the first sampling area, the first track 21 conveys the sample rack from the first sampling area to the first discharge position 213, so that the sample rack can be transferred from the first discharge position 213 of the first track 21 to the transfer track 231.

Further, the first sample convey mechanism includes a first sample delivery arm 411 and a second sample delivery arm 412 which are independent.

In this embodiment, the reaction wheel 10 includes an outer reaction ring 11 and an inner reaction ring 12 located inside the outer reaction ring 11.

The step S12 further includes aspirating, by the first sample delivery arm 411, a first sample in one of the sample containers at the shared sample aspiration position 211; transferring, by the first sample delivery arm 411, the first sample to a reaction vessel carried by the inner reaction ring 12; and aspirating and transferring, by the second sample delivery arm 412, the first sample in the one of the sample containers at the shared sample aspiration position 211 or a second sample in another one of the sample containers at the shared sample aspiration position 211 to a reaction vessel carried by the outer reaction ring 11 when the first sample is transferred by the first sample delivery arm 411 to the reaction vessel carried by the inner reaction ring 12.

During the process in which the first sample convey mechanism transfers the sample from the sample container at the shared sample aspiration position 211 to the reaction vessel, the first sample delivery arm 411 transfers the sample from the sample container at the shared sample aspiration position 211 to the reaction vessel carried by the inner reaction ring 12, and the second sample delivery arm 412 transfers the sample from the sample container at the shared sample aspiration position 211 to the reaction vessel carried by the outer reaction ring 11.

Specifically, the first sample convey mechanism includes a first sample delivery arm 411 and a second sample delivery arm 412. In case of the reaction wheel 10 with dual-ring structure, the sample needle of the first sample delivery arm 411 is used to transfer the sample taken from the shared sample aspiration position 211 to the reaction vessel carried by either the inner reaction ring 12 or the outer reaction ring 11. Similarly, the sample needle of the second sample delivery arm 412 can also transfer the sample taken from the shared sample aspiration position 211 to the reaction vessel carried by either the inner reaction ring 12 or the outer reaction ring 11.

Preferably, the sample needle of the first sample delivery arm 411 is used to transfer the sample taken from the shared sample aspiration position 211 to the reaction vessel carried by the inner reaction ring 12, and the sample needle of the second sample delivery arm 412 is used to transfer the sample taken from the shared sample aspiration position 211 to the reaction vessel carried by the outer reaction ring 11.

In a specific embodiment, the sample needle of the first sample delivery arm 411 is used to add the sample from the sample container at the shared sample aspiration position 211 to the reaction vessel carried by the inner reaction ring 12, and the sample needle of the second sample delivery arm 412 is used to add the sample from the sample container at the shared sample aspiration position 211 to the reaction vessel carried by the outer reaction ring 11.

As shown in FIG. 3, after the sample rack moves from the second sampling area to the access position 221, and then is transferred to the sample rack transfer mechanism 23, the sample transportation method further includes:

Step S70: driving, by a first driving device 232 of the sample rack transfer mechanism 23, a transfer track 231 to connect to a third feed position 241 of a third track 24.

In this step, the sample analyzer 100 further includes a third track 24, which is used for recycling sample rack. After the samples have been completely aspirated by means of the second sample dispensing mechanism from the sample rack, the sample rack transfer mechanism 23 transfers the sample rack to the third track 24, and the sample rack exits the sample analyzer 100 through the third track 24.

The first driving device 232 can drive the transfer track 231 to connect with the first discharge position 213 of the first track 21, the access position 221 of the second track 22, and the third feed position 241 of the third track 24. After the samples have been completely aspirated by means of the second sample dispensing mechanism from the sample rack, the second track 22 moves the sample rack to the transfer track 231, and then the first driving device 232 drives the transfer track 231, which carries the sample rack, to connect with the third feed position 241 of the third track 24.

Step S80: transferring the sample rack from the transfer track 231 to the third feed position 241.

In this step, after the transfer track 231 carrying the sample rack connects with the third feed position 241 of the third track 24, the transfer track 231 moves the sample rack to the third feed position 241 of the third track 24.

Step S90: transferring the sample rack from the third feed position 241 to a third discharge position 242 of the third track 24 to make the sample rack exit the sample analyzer 100.

In this step, after the transfer track 231 moves the sample rack to the third feed position 241 of the third track 24, the third track 24 moves the sample rack to exit the sample analyzer 100.

As shown in FIG. 5, in another aspect, the present invention further provides a sample transportation method for a sample analyzer 100. The sample transportation method includes the following steps:

Step 100: in a first sampling area, transferring, by a first sample dispensing mechanism, the samples requiring biochemical analyzing from a plurality of sample containers carried by a first sample rack to the plurality of reaction vessels carried by the reaction wheel 10.

In this step, the sample analyzer 100 includes a reaction wheel 10, a first track 21, and a first sample convey mechanism. The first track 21 and the first sample convey mechanism are located on the outside of the reaction wheel 10.

The first sampling area is located on the first track 21. The first sample convey mechanism transfers the sample from the sample container carried by the first sample rack in the first sampling area to the reaction vessel carried by the reaction wheel 10 for biochemical analyzing of the sample.

Step 200: in a second sampling area, transferring, by a second sample dispensing mechanism, the samples requiring electrolyte analyzing from a plurality of sample containers carried by a second sample rack to the one or more analyzing vessels carried by the electrolyte analyzing mechanism 30.

In this step, the sample analyzer 100 also includes a second track 22 and a second sample dispensing mechanism. The second track 22 and the second sample dispensing mechanism are located on the outside of the reaction wheel 10. The second sampling area is located on the second track 22.

The second sample dispensing mechanism and the first sample convey mechanism operate independently from each other, and the second sample dispensing mechanism is used to transfer the sample from the sample container carried by the second sample rack in the second sampling area to the analyzing vessel carried by the electrolyte analyzing mechanism 30 for electrolyte analyzing of the sample.

Step 300: enabling the second sample rack to pass through the first sampling area during the process of transferring the second sample rack to the second sampling area.

In this step, the second track 22 is parallel to the first track 21. The sample analyzer 100 also includes a sample rack transfer mechanism 23, which can be used to transfer the second sample rack from the first track 21 to the second track 22.

Specifically, the second sample rack enters the first track 21 via the first feed position 212 of the first track 21, then moves along the first track 21 to the first discharge position 213 of the first track 21, and finally is transferred to the sample rack transfer mechanism 23 through the first discharge position 213.

Further, in one embodiment, when the second sample rack reaches the first sampling area, the first sample convey mechanism transfers the sample from the sample container carried by the second sample rack to the reaction vessel; and when the second sample rack reaches the second sampling area, the second sample dispensing mechanism transfers the sample from the sample container carried by the second sample rack to the analyzing vessel.

Specifically, in this embodiment, the sample contained in the sample container carried by the second sample rack needs both the biochemical analyzing and the electrolyte analyzing.

Therefore, after the second sample rack moves to the first sampling area on the first track 21, the first sample convey mechanism will collect sample carried by the second sample rack for biochemical analyzing.

After the samples have been completely aspirated by means of the first sample convey mechanism, the second sample rack will be transferred to the second track 22 with the cooperation of the sample rack transfer mechanism 23, and will then move along the second track 22 to the second sampling area.

When the second sample rack reaches the second sampling area, the second sample dispensing mechanism will collect sample carried by the second sample rack for electrolyte analyzing.

In some embodiments, after the first sample convey mechanism transfers the sample from the sample container carried by the second sample rack to the reaction vessel, the second sample rack moves from the first sampling area to the first discharge position 213 of the first track 21, and is then transferred from the first discharge position 213 to the sample rack transfer mechanism 23.

Specifically, the sample rack transfer mechanism 23 includes a first driving device 232 and a transfer track 231. The first driving device 232 can drive the transfer track 231 to connect with the first discharge position 213 of the first track 21 and the access position 221 of the second track 22.

After the transfer track 231 connects with the first discharge position 213 of the first track 21, the first track 21 moves the second sample rack from the first sampling area to the first discharge position 213, and the second sample rack is transferred from the first discharge position 213 to the transfer track 231.

Subsequently, the sample rack transfer mechanism 23 transfers the second sample rack to the access position 221 of the second track 22.

After the second sample rack is transferred to the transfer track 231, the first driving device 232 drives the transfer track 231 which carries the second sample rack, to move and connect with the access position 221 of the second track 22.

After the transfer track 231 connects with the access position 221 of the second track 22, the transfer track 231 moves to convey the second sample rack to the access position 221 of the second track 22.

The second sample rack moves from the access position 221 to the second sampling area, and the second sample dispensing mechanism transfers the sample from the sample container carried by the second sample rack to the analyzing vessels carried by the electrolyte analyzing mechanism 30.

After the second sample rack is transferred to the access position 221 of the second track 22, the second track 22 continues to convey the second sample rack to the second sampling area for the second sample dispensing mechanism to sample the required sample for electrolyte analyzing.

After the second sample dispensing mechanism transfers the sample from the sample containers carried by the second sample rack to the analyzing vessels, the second sample rack moves from the second sampling area to the access position 221, and is then transferred from the access position 221 to the sample rack transfer mechanism 23.

Specifically, after the samples required for electrolyte analyzing have been completely aspirated by means of the second sample dispensing mechanism, the second sample rack will move along the second track 22 to the access position 221 of the second track 22.

When the second sample rack reaches the access position 221 of the second track 22, the second track 22 moves to transfer the second sample rack to the transfer track 231.

Furthermore, during the process in which the second sample dispensing mechanism transfers the sample from the sample containers carried by the second sample rack to the analyzing vessels, the second sample rack remains stationary in the second sampling area, and the second sample dispensing mechanism moves to different sample containers carried by the second sample rack to aspirate the samples.

In this embodiment, the sample needle of the second sample dispensing mechanism can move to sample from different sample containers carried by the second sample rack, which is stationary on the second track 22.

In one embodiment, the second sample dispensing mechanism includes a folding arm and a sample needle. The folding arm can drive the sample needle to move to different sample containers carried by the second sample rack to aspirate the samples.

Specifically, the folding arm includes at least a first folding sub-arm 422 and a second folding sub-arm 423. One end of the first folding sub-arm 422 is rotatably arranged about the first rotation center 421, and the other end of the first folding sub-arm 422 is connected to one end of the second folding sub-arm 423. The second folding sub-arm 423 can rotate relative to the first folding sub-arm 422, and the other end of the second folding sub-arm 423 is connected to the sample needle.

By providing the sample needle on the folding arm, the sample needle can sample within the second sampling area on the second track 22. The second sampling area can at least accommodate one second sample rack, so that the sample needle can sample from any sample container of the second sample rack that is stationary in the second sampling area.

In another embodiment, the second sample dispensing mechanism includes a telescopic arm and a sample needle. The telescopic arm can drive the sample needle to move to different sample containers carried by the second sample rack to take the samples.

Specifically, the telescopic arm includes at least a first telescopic sub-arm and a second telescopic sub-arm. One end of the first telescopic sub-arm is rotatably arranged about the first rotation center 421, and the other end of the first telescopic sub-arm is connected to one end of the second telescopic sub-arm. The second telescopic sub-arm is telescopic relative to the first telescopic sub-arm, and the other end of the second telescopic sub-arm is connected to the sample needle.

By providing the sample needle on the telescopic arm, the sample needle can sample within the second sampling area on the second track 22. The second sampling area can at least accommodate one second sample rack, so that the sample needle can sample from any sample container of the second sample rack that is stationary in the second sampling area.

Furthermore, during the process in which the first sample convey mechanism transfers the sample from the sample containers carried by the first sample rack to the reaction vessels, the sample containers carried by the first sample rack pass sequentially through the shared sample aspiration position 211 located in the first sampling area. The first sample convey mechanism sequentially transfers the samples from the sample containers at the shared sample aspiration position 211 into the reaction vessels.

In one embodiment, the first sample convey mechanism includes a first sample delivery arm 411 and a second sample delivery arm 412 which are independent from each other.

The reaction wheel 10 includes an outer reaction ring 11 and an inner reaction ring 12 located inside the outer reaction ring 11. The first sample convey mechanism includes the first sample delivery arm 411 and the second sample delivery arm 412, both of which sample at the shared sample aspiration position 211 in the first sampling area. The first sample rack moves along the first track 21, allowing the sample containers on the first sample rack to sequentially reach the shared sample aspiration position 211.

Specifically, the motion trajectories of the sample needles of the first sample delivery arm 411 and the second sample delivery arm 412 intersect with the first track 21 at the shared sample aspiration position 211, so that the sample needles of both the first sample delivery arm 411 and the second sample delivery arm 412 can sample at the shared sample aspiration position 211.

In case of the reaction wheel 10 with a double-ring structure, the sample needle of the first sample delivery arm 411 can be used to transfer the sample taken at the shared sample aspiration position 211 to the reaction vessel carried by either the inner reaction ring 12 or the outer reaction ring 11. The sample needle of the second sample delivery arm 412 can also be used to transfer the sample taken at the shared sample aspiration position 211 to the reaction vessel carried by either the inner reaction ring 12 or the outer reaction ring 11.

Preferably, the sample needle of the first sample delivery arm 411 is used to transfer the sample taken at the shared sample aspiration position 211 to the reaction vessel carried by the inner reaction ring 12, and the sample needle of the second sample delivery arm 412 is used to transfer the sample taken at the shared sample aspiration position 211 to the reaction vessel carried by the outer reaction ring 11.

Further, the first rotation center 421 and the second rotation center 413 are located on opposite sides of the vertical symmetry line 104, while the third rotation center 414 and the second rotation center 413 are positioned on the same side of the vertical symmetry line 104. This configuration ensures that the first and second sample dispensing mechanisms are arranged on opposite sides of the vertical symmetry line 104, thereby preventing interference between the two mechanisms when conveying biochemical sample and electrolyte sample addition.

Furthermore, the second rotation center 413 is positioned between the first rotation center 421 and the third rotation center 414. In a specific embodiment, the sample needle of the first sample delivery arm 411 is used to add the sample from the sample container at the shared sample aspiration position 211 to the reaction vessel carried by the inner reaction ring 12, while the sample needle of the second sample delivery arm 412 is used to add the sample from the sample container at the shared sample aspiration position 211 to the reaction vessel carried by the outer reaction ring 11.

For the sake of description, spatial relative terms such as "above" , "over" , "on the upper surface of" , "on top of" , etc., can be used to describe the spatial relationship between a device and other devices or between feature and other features as shown in the drawings. It should be understood that these spatial relative terms are intended to include different orientations during use or operation, in addition to the orientation described in the drawings. For example, if a device in the drawings is inverted, a device originally described as being "above" or "over" another device will then be positioned as "below" or "under" the other device. Therefore, the exemplary term "above" can include both "above" and "below" orientations. The device may also be positioned in other different ways (e.g., rotated 90 degrees or in another orientation), and the spatial relative descriptions used here should be interpreted accordingly.

In addition, it should be noted that the use of terms like "first" , "second" , etc., to define components is solely for the purpose of distinguishing the corresponding components. Unless otherwise stated, these terms do not carry any special meaning and should not be understood as limiting the scope of protection of the present invention.

The above is only a preferred embodiment of the present invention. It should be noted that for those skilled in the art, various improvements and modifications can be made without departing from the principles of the present invention. These improvements and modifications should also be considered within the scope of protection of the present invention.

## Claims

1. A sample analyzer, **characterized by** comprising:
a first track (21);
a second track (22);
a reaction wheel (10) carrying a plurality of reaction vessels;
an electrolyte analyzing mechanism (30) carrying one or more analyzing vessels;
a first sample dispensing mechanism configured to transfer samples requiring biochemical analyzing located on the first track (21) into the plurality of reaction vessels; and
a second sample dispensing mechanism configured to transfer samples requiring electrolyte analyzing located on the second track (22) into the one or more analyzing vessels.

2. The sample analyzer according to claim 1, wherein the reaction wheel (10) has a horizontal symmetry line (102) and a vertical symmetry line (104); and
the electrolyte analyzing mechanism (30), a first rotation center (421) of the second sample dispensing mechanism and the second track (22) are arranged between the first track (21) and the horizontal symmetry line (102) and are located on a same side of the vertical symmetry line (104).

3. The sample analyzer according to claim 1, wherein when the second sample dispensing mechanism transfers the samples requiring electrolyte analyzing from the second track (22) to the one or more analyzing vessels, a second sample rack remains stationary on the second track (22), and the second sample dispensing mechanism moves to different sample containers carried by the second sample rack to aspirate the samples requiring electrolyte analyzing.

4. The sample analyzer according to claim 3, wherein the second sample dispensing mechanism comprises a folding arm and a sample needle, and the folding arm is configured to drive the sample needle to move to the different sample containers carried by the second sample rack to aspirate the samples requiring electrolyte analyzing; or
wherein the second sample dispensing mechanism comprises a telescopic arm and a sample needle, and the telescopic arm is configured to drive the sample needle to move to the different sample containers carried by the second sample rack to aspirate the samples requiring electrolyte analyzing.

5. The sample analyzer according to claim 2, wherein the first sample dispensing mechanism comprises a first sample delivery arm (411) and a second sample delivery arm (412) which are independent from each other, the first sample delivery arm (411) is rotatable around a second rotation center (413), and the second sample delivery arm (412) is rotatable around a third rotation center (414); and
wherein the first rotation center (421) and the second rotation center (413) are respectively located on opposite sides of the vertical symmetry line (104), the third rotation center (414) and the second rotation center (413) are located on a same side of the vertical symmetry line (104), and the second rotation center (413) is located between the first rotation center (421) and the third rotation center (414).

6. The sample analyzer according to claim 5, wherein the reaction wheel (10) comprises an outer reaction ring (11), and an inner reaction ring (12) located on an inner side of the outer reaction ring (11);
wherein the first sample delivery arm (411) is configured to transfer the samples requiring biochemical analyzing located on the first track (21) into reaction vessels carried by the inner reaction ring (12), and the second sample delivery arm (412) is configured to transfer the samples requiring biochemical analyzing located on the first track (21) into reaction vessels carried by the outer reaction ring (11); and
wherein both the first sample delivery arm (411) and the second sample delivery arm (412) perform aspiration at a shared sample aspiration position (211) on the first track (21), and a first sample rack moves along the first track (21) to make a plurality of sample containers on the first sample rack sequentially reach the shared sample aspiration position (211).

7. The sample analyzer according to claim 2, wherein the reaction wheel (10) comprises a first reagent dispensing position (13) and a second reagent dispensing position (14);
the sample analyzer further comprises:
a reagent container storage mechanism configured to carry a plurality of reagent containers, wherein the reagent container storage mechanism is located on an outside of the reaction wheel (10) and on a side of the horizontal symmetry line (102) away from the electrolyte analyzing mechanism (30), and the reagent container storage mechanism comprises a first reagent aspirating position (51) and a second reagent aspirating position (52); and
a reagent injection mechanism comprising a first reagent needle (61) and a second reagent needle which are independently controlled, wherein the first reagent needle (61) is configured to draw a reagent from one of the plurality of reagent containers that is located at the first reagent aspirating position (51), transfer the reagent along a first straight line, and inject the reagent into one of the plurality of reaction vessels that is located at the first reagent dispensing position (13) at a time, and the second reagent needle is configured to draw a reagent from one of the plurality of reagent containers that is located at the second reagent aspirating position (52), transfer the reagent along a second straight line, and inject the reagent into one of the plurality of reaction vessels that is located at the second reagent dispensing position (14) at a time.

8. The sample analyzer according to claim 1, wherein samples located on the first track (21) and samples located on the second track (22) enter the sample analyzer via the first track (21);
a sample only to be transferred to one of the plurality of reaction vessels is received by a sample container carried by a first sample rack;
a sample at least to be transferred to the analyzing vessel or one of the analyzing vessels is received by a sample container carried by a second sample rack; and
the sample analyzer further comprises a sample rack transfer mechanism (23), and the second sample rack is transferable from the first track (21) to the second track (22) by the sample rack transfer mechanism (23).

9. The sample analyzer according to claim 8, further comprising a third track (24), wherein:
the first track (21), the second track (22) and the third track (24) are arranged parallel to each other, and the first sample rack and the second sample rack exit the sample analyzer via the third track (24);
the sample rack transfer mechanism (23) is further configured to transfer the first sample rack on the first track (21) or the second sample rack on the second track (22) to the third track (24); and
wherein the sample rack transfer mechanism (23) comprises a transfer track (231) and a first driving device (232), and the first driving device (232) is configured to drive the transfer track (231) to connect to a first discharge position (213) of the first track (21) and further configured to drive the transfer track (231) to connect to either an access position (221) of the second track (22) or a third feed position (241) of the third track (24); or
wherein the sample rack transfer mechanism (23) comprises a second driving device and a gripper, and wherein the gripper driven by the second driving device is configured to transfer the second sample rack on the first track (21) to the second track (22); and transfer the second sample rack on the second track (22) or the first sample rack on the first track (21) to the third track (24).

10. The sample analyzer according to claim 8, wherein the first track (21) is configured to transfer both the first sample rack and the second sample rack, the first sample rack carries samples that require biochemical analyzing, and the second sample rack carries samples that require electrolyte analyzing only or require both electrolyte analyzing and biochemical analyzing; and
the second sample rack is transferable from the first track (21) to the second track (22) and carries samples that require electrolyte analyzing.

11. A sample aspiration method for a sample analyzer, **characterized by** comprising steps of:
in a first sampling area, transferring, by a first sample dispensing mechanism, samples requiring biochemical analyzing from a plurality of sample containers carried by a first sample rack to a plurality of reaction vessels carried by a reaction wheel (10); and
in a second sampling area, transferring, by a second sample dispensing mechanism, samples requiring electrolyte analyzing from a plurality of sample containers carried by a second sample rack to one or more analyzing vessels carried by a electrolyte analyzing mechanism (30),
wherein before the step of transferring samples requiring electrolyte analyzing from a plurality of sample containers carried by a second sample rack to one or more analyzing vessels carried by a electrolyte analyzing mechanism (30), the method further comprises transferring the second sample rack to the second sampling area, during which the second sample rack passes through the first sampling area.

12. The sample aspiration method for the sample analyzer according to claim 11, wherein the first sampling area is located on a first track (21), the second sampling area is located on a second track (22), and the second track (22) is parallel to the first track (21);
the method further comprises steps of:
transferring the second sample rack from the first track (21) to a sample rack transfer mechanism (23); and
transferring the second sample rack from the sample rack transfer mechanism (23) to the second track (22).

13. The sample aspiration method for the sample analyzer according to claim 12, further comprises steps of:
transferring, by the first sample dispensing mechanism, the samples requiring biochemical analyzing from the plurality of sample containers carried by the second sample rack to the plurality of reaction vessels when the second sample rack reaches the first sampling area; and
transferring, by the second sample dispensing mechanism, the samples requiring electrolyte analyzing from the plurality of sample containers carried by the second sample rack to the one or more analyzing vessels when the second sample rack reaches the second sampling area;
wherein after the step of transferring, by the first sample dispensing mechanism, the samples requiring biochemical analyzing from the plurality of sample containers carried by the second sample rack to the plurality of reaction vessels, the method further comprises:
transferring the second sample rack from the first sampling area to a first discharge position (213) of the first track (21), and then from the first discharge position (213) to the sample rack transfer mechanism (23);
transferring, by the sample rack transfer mechanism (23), the second sample rack to an access position (221) of the second track (22);
transferring, the second sample rack from the access position (221) to the second sampling area, that is followed by the step of transferring, by the second sample dispensing mechanism, the samples requiring electrolyte analyzing from the plurality of the sample containers carried by the second sample rack to the one or more analyzing vessels carried by the electrolyte analyzing mechanism (30); and
transferring the second sample rack from the second sampling area to the access position (221), and then from the access position (221) to the sample rack transfer mechanism (23) after the step of transferring, by the second sample dispensing mechanism, the samples requiring electrolyte analyzing from the plurality of sample containers carried by the second sample rack to the one or more analyzing vessels.

14. The sample aspiration method for the sample analyzer according to claim 11, wherein during the step of transferring, by a second sample dispensing mechanism, samples requiring electrolyte analyzing from a plurality of the sample containers carried by a second sample rack to one or more analyzing vessels, the second sample rack remains stationary in the second sampling area, and the second sample dispensing mechanism is capable of moving to different sample containers carried by the second sample rack to aspirate the samples requiring electrolyte analyzing.

15. The sample aspiration method for the sample analyzer according to claim 11, wherein during the step of transferring, by a first sample dispensing mechanism, samples requiring biochemical analyzing from a plurality of sample containers carried by a first sample rack to a plurality of reaction vessels, the plurality of sample containers carried by the first sample rack pass sequentially through a shared sample aspiration position (211), and the first sample dispensing mechanism sequentially transfers the samples requiring biochemical analyzing from the plurality of sample containers located at the shared sample aspiration position (211) to the plurality of reaction vessels;
wherein the first sample dispensing mechanism comprises a first sample delivery arm (411) and a second sample delivery arm (412) which are independent from each other; and
the reaction wheel (10) comprises an outer reaction ring (11) and an inner reaction ring (12) located on an inner side of the outer reaction ring (11); and
wherein the step of transferring, by the first sample dispensing mechanism, the samples requiring biochemical analyzing from a plurality of sample containers at the shared sample aspiration position (211) to the plurality of reaction vessels comprises steps of:
aspirating, by the first sample delivery arm (411), a first sample in one of the plurality of sample containers at the shared sample aspiration position (211);
transferring, by the first sample delivery arm (411), the first sample to one of the plurality of reaction vessels carried by the inner reaction ring (12); and
aspirating and transferring, by the second sample delivery arm (412), the first sample in the one of the plurality of sample containers at the shared sample aspiration position (211) or a second sample in another one of the plurality of sample containers at the shared sample aspiration position (211) to one of the plurality of reaction vessels carried by the outer reaction ring (11) when the first sample is transferred by the first sample delivery arm (411) to one of the plurality of reaction vessels carried by the inner reaction ring (12).
